(19) Europäisches Patentamt
European Patent Office
Office européen des brevets

(11) Publication number: 0 003 847 A1

# (12) EUROPEAN PATENT APPLICATION

(21) Application number: 79100555.6

(22) Date of filing: 23.02.79

(51) Int. Cl.[2]: C 07 C 101/72
C 07 C 125/06

(30) Priority: 27.02.78 JP 20906/78

(43) Date of publication of application: 05.09.79 Bulletin 79/18

(84) Designated contracting states: CH DE FR GB IT

(71) Applicant: Chugai Seiyaku Kabushiki Kaisha
5-1, 5-chome, Ukima Kita-ku
Tokyo(JP)

(72) Inventor: Oi, Nobuhiro
2-8-14, Nakamachi
Hoya-shi Tokyo(JP)

(72) Inventor: Aoki, Bunya
4-2-12-407, Nagayama
Tama-shi Tokyo(JP)

(72) Inventor: Shinozaki, Teizo
2-25, Tokiwadaira
JP(JP)

(72) Inventor: Moro, Kanji
2-11-9, Kurihara
Kuki-shi Saitama-Ken(JP)

(72) Inventor: Matsunaga, Isao
3140-20, Nishi Ohizumicho
Nerima-ku Tokyo(JP)

(72) Inventor: Shindo, Minoru
4-10-7, Nukui
Nerima-ku Tokyo(JP)

(74) Representative: Vossius, Volker, Dr. et al,
Vossius, Vossius, Hiltl, Tauchner, Heunemann
Patentanwälte Siebertstrasse 4
D-8000 München 86(DE)

## (54) Biphenylylglycine derivatives and their salts with bases.

(57) Biphenylylglycine derivatives of the general formula I

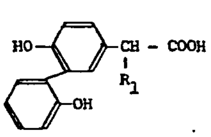

(1)

wherein R1 is an amino group or a protected amino group and their salts with bases. The compounds are useful as intermediates for synthesizing various penicillin and cephalosporin derivatives which have excellent antibacterial activities. The compounds can be prepared, for example, by reacting an N-substituted-α-hydroxy-glycine with o,o'- dihydroxybiphenyl in the presence of an acid catalyst in an inert organic solvent. The compounds contain an asymmetric carbon atom at the α-position to the carboxy group. The invention covers the racemic mixtures and the D- and L-isomers.

EP 0 003 847 A1

0003847

VOSSIUS · VOSSIUS · HILTL · TAUCHNER · HEUNEMANN
PATENTANWÄLTE
MÜNCHEN

Our Ref.: P 032 EP
Case: FP(EPC)/C-1-403

2 8. Feb. 1979

CHUGAI SEIYAKU KABUSHIKI KAISHA
Tokyo, Japan

---

" Biphenylylglycine Derivatives and their Salts with Bases "

---

Priority: February 27, 1978, Japan, No. 20906/78

---

This invention relates to biphenylylglycine derivatives of the general formula I

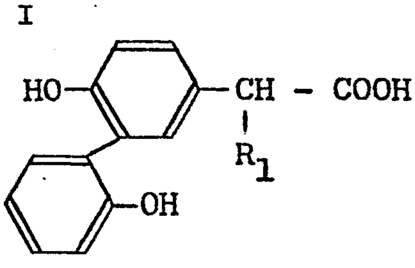

(I)

wherein $R_1$ is an amino group or a protected amino group and their salts with bases.

The term "protected amino group" used herein refers to an amino group, at least one hydrogen atom of which is substituted by a group capable of being easily split off, or an amino group to which a group is added which is capable of being easily split off. This includes an amino group protected by any group which is usually used as a so-called protecting group in the field of amino acid or peptide synthesis.

Examples of the groups which can be used in this invention are substituted or unsubstituted benzylidene groups, substituted alkyl or alkenyl groups such as the triphenylmethyl group or 1-methoxycarbonyl-1-propene-2-yl group; mercapto groups such as the 2-nitrophenylmercapto group; organic or inorganic acids such as hydrochloric acid or benzene sulfonic acid; acyl groups of the general formula II

$$-CO-R_2 \qquad (II)$$

(wherein $R_2$ is a hydrogen atom; a substituted or unsubstituted lower alkyl group, for example, methyl, ethyl, propyl, n-butyl, iso-butyl, tert.-butyl, trifluoromethyl or 2,2,2-trihaloethyl; an aralkyl group, for example, benzyl or α-phenethyl; or an aryl group, for example, phenyl or naphthyl. Said aralkyl group or aryl group may be substituted by one or more groups selected from halogen atoms, a hydroxyl group, a lower alkyl group, a lower alkoxy group or a nitro group) or a group represented by the general formula III

$$-CO-Y-R_3 \qquad (III)$$

(wherein Y is an oxygen atom, a sulfur atom or an imino group, $R_3$ is a lower alkyl group, for example, methyl, ethyl, propyl, n-butyl, iso-butyl, tert.-butyl or a halogen-substituted lower alkyl group such as 2,2,2-trihaloethyl; an aralkyl group, for example, benzyl or α-phenethyl; an aryl group, for example, phenyl or naphthyl, said aralkyl or aryl group which can be substituted by one or more groups selected from the group consisting of a halogen atom, a hydroxyl group, a lower alkyl group, a lower alkoxyl group and a nitro group; an adamantyl group; or a hydrogen atom when Y is an imino group), among which a group represented by the general formula IV

$$-CO-O-R_4 \qquad (IV)$$

(wherein $R_4$ is a lower alkyl group, for example, tert.-butyl; a lower halo-alkyl group, for example, 2,2,2-trihaloethyl; a substituted or unsubstituted benzyl group, for example, 4-methoxy-benzyl or benzyl; or an adamantyl group) is preferred.

The term "a lower alkyl group" or "a lower alkoxy group" means a group containing 1 to 5 carbon atoms, preferably 1 or 2 carbon atoms.

The salts of biphenylylglycine derivatives represented by the general formula (I) include salts with an alkali metal such as sodium or potassium; an alkaline earth metal such as calcium or barium; and an organic base, for example, tertiary amines such as triethylamine, N-methylpiperidine and pyridine.

The biphenylylglycine derivatives of the general formula (I) contain an asymmetric carbon atom at the α-position to the carboxy group. Therefore, the compounds can exist as optical isomers, namely D- and L-isomers. All such isomers and the racemic mixtures are included in this invention.

The compounds of the general formula (I) are prepared, for example, by the following method.

An N-protected-α-hydroxyglycine is reacted with o,o'-dihydroxybiphenyl in the presence of an acid catalyst in an inert organic solvent. Examples of the acid catalyst which are useful in this reaction are Lewis acids such as boron trifluoride ethyl etherate and aluminium chloride; mineral acids such as sulphuric acid; and organic acids such as formic acid and trifluoroacetic acid.

The inert organic solvents which may be used in the reaction include acetic acid, ethyl acetate, dioxane and tetrahydrofuran.

The reaction proceeds at room temperature, but may be controlled by elevating or lowering the reaction temperature, if desired. The isolation of the product from the reaction mixture may be effected by any conventional technique such as extraction with ethyl acetate.

The compounds of the general formula (I) wherein $R_1$ is an amino group can be formed by removing the protecting group from the N-protected biphenylylglycine which has been prepared by the above method. The removal of the protecting group may be accomplished by conventional techniques, depending on the particular protecting group. Conventional techniques include, for example, reduction, treatment with a strong organic acid such as trifluoroacetic acid, formic acid, etc..

The product is obtained as a racemic mixture and, if desired, it can be subjected to resolution to isolate an optically active compound (enantiomer). The resolution can be accomplished by any suitable conventional technique. An example of such a technique comprises forming a salt of the product with an optically active base such as brucine, strychnine, ephedrine, quinine or similar and then fractionally crystallizing the resulting

diastereomeric salt. In certain cases resolution can be effected by using an optically active acid such as malic acid, tartaric acid or similar, after esterifying the carboxyl group of the product.

Furthermore, the optically active compounds may be isolated from the racemic mixture by utilizing an asymmetric inductive effect. That is, the resolution can be accomplished by bonding an optically active group as a protecting group, e.g. an α-phenylethyloxycarbonyl group, to the amino group of the objective compound in the form of a racemic mixture and by fractionally crystallizing the protected compound.

The compounds of the general formula (I) or their salts prepared in the manner described above are useful as intermediates for synthesizing various penicillin derivatives and cephalosporin derivatives which have excellent antibacterial activities. For example, 7β-[DL-α-amino-α-{3-(2-hydroxyphenyl)-4-hydroxyphenyl}acetamido]-desacetoxycephalosporin which has strong antibacterial activities against both gram-positive and gram-negative bacteria is prepared from one of the object compounds of this invention, DL-α-{3-(2-hydroxyphenyl)-4-hydroxyphenyl}glycine and 7-aminodesacetoxy-cephalosporanic acid.

The processes for preparing some of the compounds of this invention are further illustrated by the following examples:

### Example 1

Glyoxalic acid hydrate (9.2 g; 0.1 moles), tert.-butyl carbonate (11.7 g; 0.1 moles) and 2,2'-dihydroxybiphenyl (37.2 g; 0.2 moles) were dissolved in ethyl acetate (75 ml). Boron trifluoride ethyl etherate (1.0 ml) was added to the solution under cooling with ice-water and

the mixture was allowed to stand at 25°C for 48 hours. The reaction mixture was treated with a saturated aqueous sodium bicarbonate solution to adjust its pH-value to 7.5 and the aqueous layer was recovered. The layer was washed three times with ethyl acetate (total volume 300 ml). The separated aqueous layer was thoroughly cooled with ice-water and, after adjusting its pH-value to 4.0 with 2N hydrochloric acid, was extracted with ethyl acetate (100 ml). The separated organic layer was dried over anhydrous sodium sulphate and concentrated. To the residue was added cold ethyl acetate (30 ml) and, after removing undissolved solid by filtration, the filtrate was concentrated. The residue was triturated in chloroform and recovered. The product was recrystallized from a mixture of ethyl acetate and chloroform to give 16.0 g of DL-N-tert.-butoxycarbonyl-α-{3-(2-hydroxyphenyl)-4-hydroxyphenyl}glycine (Yield: 44 %), m.p. 203 - 205°C (decomposition).

Analysis:
Calcd. for $C_{19}H_{21}NO_6$: C, 63.50; H, 5.89; N, 3.90 (%)
Found : C, 63.21; H, 5.72; N, 4.20 (%)

Example 2

N-Benzyloxycarbonyl-α-hydroxyglycine (100 g; 0.44 moles) and o,o'-dihydroxybiphenyl (166 g; 0.89 moles) were suspended in glacial acetic acid (600 ml) and to the suspension was added 98 % sulphuric acid (60 ml) under cooling with ice water. After stirring the mixture at room temperature for 48 hours, it was poured into ice-water (3 litres). Ethyl acetate (500 ml) was added to the mixture and, after shaking, the ethyl acetate layer was recovered. This extraction was repeated with another 500 ml of ethyl acetate, and the recovered ethyl acetate layers were combined and then washed with a saturated aqueous sodium chloride solution. The ethyl acetate

layer was extracted three times with a total amount of 600 ml of a saturated aqueous sodium bicarbonate solution. The aqueous extracts were combined, cooled thoroughly with ice-water and the pH-value adjusted to 4.0 with 2N hydrochloric acid. The aqueous layer was extracted three times with a total amount of 1 litre of ethyl acetate and the extracts were combined, washed with a saturated aqueous sodium chloride solution, dried over anhydrous sodium sulphate and concentrated. After adding hot chloroform (800 ml) to the residue and allowing the mixture to stand overnight, the resulting solid was recovered and recrystallized from a mixture of ethyl acetate and chloroform to give 216 g of DL-N-benzyloxycarbonyl-α-{3-(2-hydroxyphenyl)-4-hydroxyphenyl}glycine (Yield: 55 %), m.p. 173°C.

Analysis:

| | |
|---|---|
| Calcd. for $C_{22}H_{19}NO_6$: | C, 67.17; H, 4.87; N, 3.56 (%) |
| Found : | C, 66.97; H, 4.82; N, 3.53 (%) |

## Example 3

DL-N-Benzyloxycarbonyl-α-{3-(2-hydroxyphenyl)-4-hydroxyphenyl}glycine (58 g; 0.15 moles) obtained in Example 2 was dissolved in ethanol by heating. Quinine trihydrate (48 g; 0.15 moles) was added to the solution and dissolved. The solution was concentrated under reduced pressure and, after adding hot benzene (300 ml), the mixture was allowed to stand overnight at 5°C. The resulting quinine salt was recovered by filtration. After recrystallizing three times from ethanol-benzene, it had a melting point of 196 - 198°C (decomposition) and a specific rotation $[\alpha]_D^{25}$ of -135° (c = 1; methanol).

The quinine salt (27 g; 0.038 moles) obtained above was suspended in ethyl acetate (300 ml) and to the suspension was added a cold aqueous solution (200 ml) containing 3 g (0.075 moles) of sodium hydroxide while stirring under cooling with ice-water. After stirring the mixture at that temperature for 10 minutes, the aqueous layer was recovered, its pH-value adjusted to 2 - 3 with 2N hydrochloric acid and extracted with ethyl acetate. The extract was washed with a saturated aqueous sodium chloride solution and dried over anhydrous sodium sulphate and then the solvent was distilled off under reduced pressure. The resulting oily residue was dissolved in cold methanol (200 ml) and subjected to catalytic reduction with the addition of 5 % palladium on carbon (2 g). After completion of the reduction, the solvent was distilled off under reduced pressure. The residue was treated with ethyl acetate to give a white solid. The recrystallization of the solid from aqueous dioxane gave 11.0 g of D(-)-α-{3-(2-hydroxyphenyl)-4-hydroxyphenyl}glycine (Yield: 90 % based on the quinine salt), m.p. 99 - 100°C (decomposition), $[\alpha]_D^{25}$ = -75° (c = 1, N hydrochloric acid).

Analysis:
Calcd. for $C_{14}H_{13}NO_4 \cdot 2H_2O$: C, 56.94; H, 5.80; N, 4.74(%)
Found : C, 56.63; H, 5.93; N, 4.51(%)

What is claimed is:

1. Biphenylylglycine derivatives of the general formula I

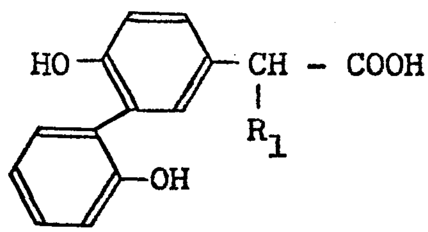

(I)

wherein $R_1$ is an amino group or a protected amino group and their salts with bases.

2. Compounds according to claim 1 wherein said protected amino group is an amino group, at least one hydrogen atom of which is substituted by a group selected from the class consisting of a substituted or unsubstituted benzylidene group; a substituted alkyl or alkenyl group; a mercapto group; an organic or inorganic acid; an acyl group of the general formula II

$$-CO-R_2 \quad (II)$$

(wherein $R_2$ is a hydrogen atom; a substituted or unsubstituted lower alkyl group; an aralkyl group; an aryl group; or an aralkyl group or aryl group substituted with one or more groups selected from a halogen atom, a hydroxyl group, a lower alkyl group, a lower alkoxyl group or a nitro group)and a group represented by the general formula III

$$-CO-Y-R_3 \quad (III)$$

(wherein Y is an oxygen atom, a sulphur atom or an

imino group, $R_3$ is a lower alkyl group; a halogen-substituted lower alkyl group; an aralkyl group; an aryl group; an aralkyl or aryl group substituted with one or more groups selected from the class consisting of a halogen atom, a hydroxyl group, a lower alkyl group, a lower alkoxyl group and a nitro group; an adamantyl group; or a hydrogen atom when Y is an imino group).

3. Compounds according to claim 2 wherein said group is selected from the class consisting of groups represented by the general formula IV

$$-CO-O-R_4 \qquad (IV)$$

(wherein $R_4$ is a lower alkyl group; a lower haloalkyl group; a substituted or unsubstituted benzyl group or an adamantyl group.)

0003847

European Patent Office

# EUROPEAN SEARCH REPORT

Application number
EP 79 10 0555

## DOCUMENTS CONSIDERED TO BE RELEVANT

| Category | Citation of document with indication, where appropriate, of relevant passages | Relevant to claim |
|---|---|---|
| A | GB - A - 1 521 419 (BRISTOL-MEYERS CO.) * Page 8, lines 37-48; page 12, line 1 - page 13, line 32; page 16, line 9 - page 19, line 13 * ---- | 1-3 |

CLASSIFICATION OF THE APPLICATION (Int. Cl.²)

C 07 C 101/72
125/06

TECHNICAL FIELDS SEARCHED (Int.Cl.²)

C 07 C 101/72
125/06

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 18-05-1979 | PAUWELS |

EPO Form 1503.1 06.78